# EUROPEAN PATENT SPECIFICATION

(11) **EP 3 740 260 B1**
(45) Date of publication and mention of the grant of the patent: **26.02.2025**
(21) Application number: 19887122.0
(22) Date of filing: 21.11.2019
(51) Int. Cl.: A61M 1/00, A61B 18/14

(54) **APPARATUS FOR FLOW**
VORRICHTUNG FÜR DURCHFLUSS
DISPOSITIF POUR ÉCOULEMENT

(30) Priority: 21.11.2018 US 201862770341 P
(43) Date of publication of application: 25.11.2020
(73) Proprietor: Buffalo Filter LLC, Lancaster, NY 14086 (US)
(72) Inventor: MILLER, Michael, New York 14043 (US); SHVETSOV, Kyrylo, New York 14043 (US); PEPE, Gregory, New York 14086 (US); BONANO, Samantha, New York 14221 (US)
(74) Representative: Flügel Preissner Schober Seidel
(86) International application number: PCT/US2019/062645
(87) International publication number: WO 2020/106977

(56) References cited:
- EP-A1- 2 946 738
- EP-B1- 2 946 738
- WO-A1-2016/144804
- US-A- 5 013 300
- US-A- 5 830 214
- US-A1- 2002 019 631
- US-A1- 2012 259 357
- US-A1- 2015 119 914
- US-A1- 2015 257 816
- US-A1- 2016 157 920

## Description

### BACKGROUND OF THE INVENTION

### FIELD OF THE INVENTION

Exemplary embodiments of the present disclosure relate to an apparatus for surgical procedures. Exemplary embodiments of the present disclosure relate more particularly to an apparatus for evacuation during surgical procedures.

### DESCRIPTION OF RELATED ART

Surgical smoke and aerosol, or plume, is often created during the performance of various types of surgeries. For example, when laser or electrosurgical energy is delivered to a cell, heat is created. This heat vaporizes the intracellular fluid, which increases the pressure inside the cell and eventually causes the cell membrane to burst. In this example, a plume of smoke containing water vapor is released into the atmosphere of the operating room, doctor's office, or other location in which the surgery is taking place. At the same time, the heat created may char the protein and other organic matter within the cell and may cause thermal necrosis in adjacent cells. The charring of cells may also release other harmful contaminants, such as carbonized cell fragments and gaseous hydrocarbons.

EP 2 946 738 A1 discloses an electrosurgical dissection instrument which includes a housing, a vacuum cannula extending distally from the housing, and an electrode extending distally from the housing through the vacuum cannula. The vacuum cannula and electrode are configured for independent, selective positioning along a longitudinal axis of the housing. An aspiration tube may be coupled to the vacuum cannula. The vacuum cannula and/or aspiration tube include one or more pre-aspiration ports and/or an elastomeric or rigid aspiration tip at a distal end thereof. A second independently-positionable vacuum cannula and/or aspiration tube may be disposed within the first vacuum cannula and/or aspiration tube. Once positioned, the vacuum cannula and electrode may be fixed in position to prevent undesired movement during use.

US 5 830 214 A discloses an electrosurgical device and method for cauterizing tissue and evacuating fluid from the surgical site. The device comprises an insulated conductive shaft having a proximal end coupled to an electric generator and a distal end coupled to an electrode for cutting or coagulating tissue. The shaft has an inner lumen fluidly coupled to a vacuum source and a plurality of insulated side holes in communication with the lumen. The side holes are configured to remain free of obstructions so that fluid, such as smoke, can be continuously evacuated during the surgical procedure.

### BRIEF SUMMARY OF THE INVENTION

In view of the foregoing, it is an object of the present disclosure to provide an apparatus for surgical procedures and a method for providing the apparatus. The apparatus in accordance with the invention is defined by claim 1. The method in accordance with the invention is defined by claim 15. Preferred embodiments of the invention are defined by claims 2 to 14, and 16.

A first exemplary embodiment of the present disclosure provides a surgical apparatus. The surgical apparatus includes an elongated body including a longitudinal axis, a distal end, and a proximal end, the elongated body including a fluid conduit extending through the longitudinal axis, and a fluid inlet disposed adjacent to the distal end, wherein the fluid inlet is operable to allow a flow of fluid at a first flow rate. The surgical apparatus further includes a cutting element disposed adjacent to the distal end, a fluid outlet disposed adjacent to the proximal end, wherein the fluid inlet and the fluid outlet are in fluid communication via the fluid conduit, and at least one perforation defined by the elongated body adjacent to the distal end, wherein the at least one perforation is in fluid communication with the fluid conduit.

A second exemplary embodiment of the present disclosure presents a surgical apparatus. The surgical apparatus includes an elongated body having a proximal end and a distal end, and a fluid inlet disposed adjacent to the distal end. The surgical apparatus further includes a cutting element disposed adjacent to the distal end, a fluid outlet disposed adjacent to the proximal end, wherein the fluid inlet and the fluid outlet are in fluid communication via a fluid conduit, and a plurality of perforations defined by the elongated body located adjacent the distal end, wherein the plurality of perforations are in fluid communication with the fluid conduit.

A third exemplary embodiment of the present disclosure presents a method of providing a surgical apparatus, the surgical apparatus including an elongated body having a proximal end and a distal end, and a fluid inlet disposed adjacent to the distal end. The surgical apparatus further includes a cutting element disposed adjacent to the distal end, and a fluid outlet disposed adjacent to the proximal end, wherein the fluid inlet and the fluid outlet are in fluid communication via a fluid conduit. The surgical apparatus still further includes at least one perforation defined by the elongated body adjacent to the distal end, wherein the at least one perforation is in fluid communication with the fluid conduit. The method further includes providing a vacuum source in fluid communication with the fluid outlet, and cutting tissue, whereby a plume develops and is at least partially communicated to the fluid conduit via the fluid inlet and the at least one perforation.

A fourth exemplary embodiment of the present disclosure presents a surgical apparatus. The surgical apparatus includes an elongated body having a proximal end and a distal end, and a fluid inlet disposed adjacent to the distal end. The surgical apparatus further includes a cutting element disposed adjacent to the distal end, and a fluid outlet disposed adjacent to the proximal end, wherein the fluid inlet and the fluid outlet are in fluid communication via a fluid conduit. The surgical apparatus still further includes a plurality of perforations defined by the elongated body located adjacent to the proximal end, wherein the plurality of perforations is in fluid communication with the fluid conduit.

The following will describe embodiments of the present disclosure, but it should be appreciated that the present disclosure is not limited to the described embodiments and various modifications of the invention are possible without departing from the basic principles. The scope of the present disclosure is therefore to be determined solely by the appended claims.

### BRIEF DESCRIPTION OF THE SEVERAL VIEWS OF THE DRAWING(S)

FIG. 1 presents a perspective view of a first embodiment of a device suitable for performing exemplary embodiments of the present disclosure.
FIG. 2 presents a perspective view of a second embodiment of the device suitable for performing exemplary embodiments of the present disclosure.
FIG. 3 presents a perspective view of a third embodiment of the device suitable for performing exemplary embodiments of the present disclosure.
FIG. 4 presents another perspective view of the third embodiment of the device suitable for performing exemplary embodiments of the present disclosure.
FIG. 5 presents a perspective view of a fourth embodiment of the device suitable for performing exemplary embodiments of the present disclosure.
FIG. 6 presents another perspective view of the fourth embodiment of the device suitable for performing exemplary embodiments of the present disclosure.
FIG. 7 presents a cross-sectional view of the exemplary device suitable for performing exemplary embodiments of the present disclosure.
FIG. 8 presents another cross-sectional view of the exemplary device suitable for performing exemplary embodiments of the present disclosure.
FIG. 9 presents a perspective view of a fifth embodiment of the device suitable for performing exemplary embodiments of the present disclosure.
FIG. 10 presents a perspective view of a sixth embodiment of the device suitable for performing exemplary embodiments of the present disclosure.
FIG. 11 presents a perspective view of a seventh embodiment of the device suitable for performing exemplary embodiments of the present disclosure.
FIG. 12 presents a perspective view of an eighth embodiment of the device suitable for performing exemplary embodiments of the present disclosure.
FIG. 13 presents a perspective view of a ninth embodiment of the device suitable for performing exemplary embodiments of the present disclosure.
FIG. 14 presents a perspective view of a tenth embodiment of the device suitable for performing exemplary embodiments of the present disclosure.
FIG. 15 presents a perspective view of an eleventh embodiment of the device suitable for performing exemplary embodiments of the present disclosure.
FIG. 16 presents a perspective view of a twelfth embodiment of the device suitable for performing exemplary embodiments of the present disclosure.
FIG. 17 presents a close-up view of the distal end of a device suitable for performing exemplary embodiments of the present disclosure.
FIG. 18 presents a logic flow diagram suitable for practicing exemplary embodiments of the present disclosure.

### DETAILED DESCRIPTION OF THE INVENTION

During some surgical procedures, plume or smoke is created near the surgical site. This plume or smoke can include gases, fluids, and/or particulates. These gases, fluids, and/or particulates can be harmful to humans if inhaled. These gases, fluids, and/or particulates can also obstruct a medical professional's view of the surgical site during the performance of the surgery. If the medical professional is unable to properly view the surgical site, the medical professional may be more inclined to make mistakes during the procedure. Accordingly, there is a need to effectively and efficiently remove surgical smoke, plume, gas, fluid, and/or particulates from and around the surgical site during the performance of the surgical procedure.

Embodiments of the present disclosure provide a surgical apparatus operable to have a flow of gas, fluid, and/or particulate pass through it. Embodiments of the present disclosure allow gas, fluid, and/or particulates created during a surgical procedure to be removed or evacuated from nearby the surgical site. Embodiments of the present disclosure provide a surgical apparatus having an increased surface area such that the surgical apparatus is operable to evacuate gas, fluid, and/or particulates from a larger area other than simply adjacent to the surgical site. Embodiments of the present disclosure provide a surgical apparatus having one or a plurality of perforations located adjacent to a cutting element, wherein each one of the one or plurality of perforations are fluidly connected to a conduit within the surgical apparatus for evacuating gas, fluid, and/or particulates. Embodiments of the present disclosure provide a surgical apparatus having a plurality of perforations disposed through the long axis of the surgical apparatus operable for allowing a flow of gas, fluid and/or particulates through the long axis of the surgical apparatus.

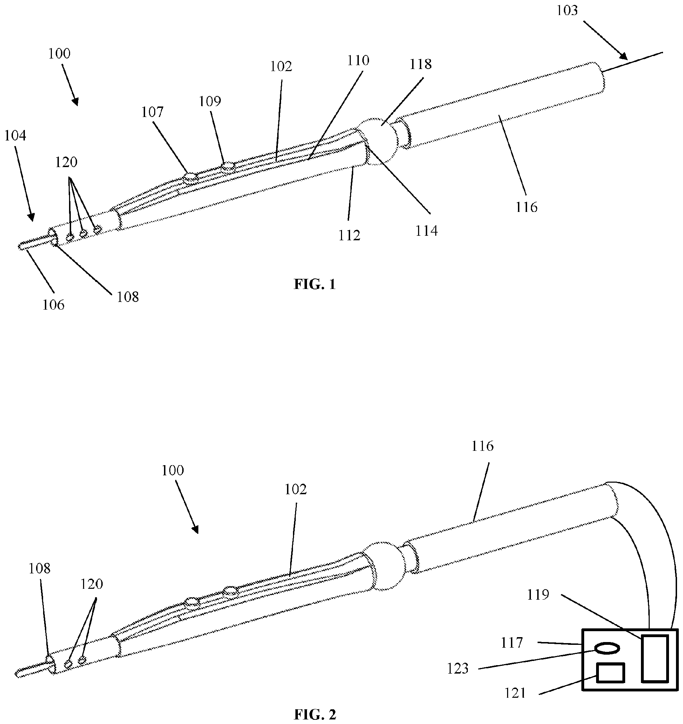

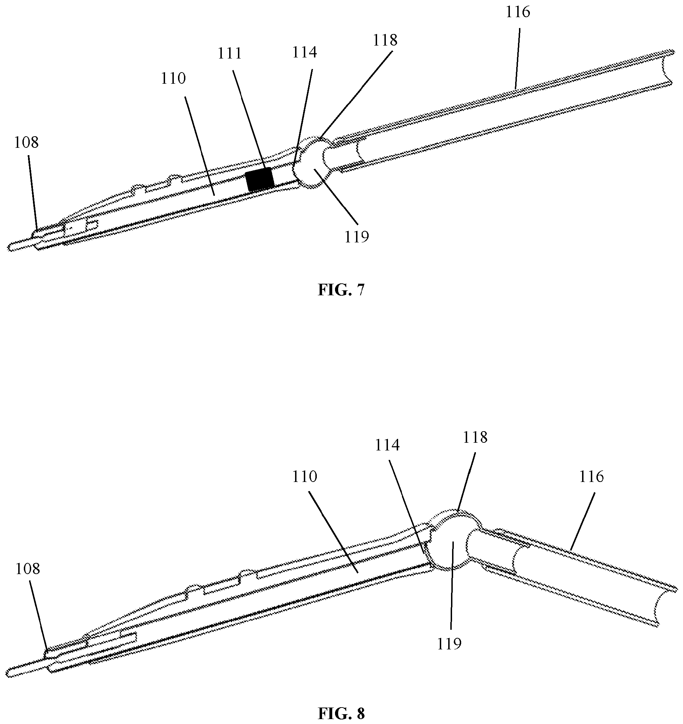

Referring to FIGs. 1-16, shown are exemplary embodiments of a device 100 suitable for performing exemplary embodiments of the present disclosure. Shown in FIG. 1 is device 100 having a body 102 and a longitudinal axis 103. At the distal end 104 of device 100 is cutting element 106. Embodiments of cutting element 106 include an electrode operable to apply an electrical current to a surgical site for cutting and/or coagulation. Embodiments of cutting element 106 include an ultrasonic scalpel or a laser scalpel. Embodiments of device 100 include a first button 107 and in some instances a second button 109. Embodiments of device 100 provide that buttons 107, 109 may be operable to (i) activate/deactivate cutting element 106, (ii) change the cutting configuration of cutting element 106 (e.g., cutting to coagulation), (iii) activate/deactivate an evacuation source, and/or (iv) operate a valve 111 (shown in FIG. 7) within conduit 110 (shown in FIG. 7 and FIG. 8) operable to allow a flow of fluid, gas and/or particulates through conduit 110 in one configuration and obstruct the flow of fluid, gas, and/or particulates through conduit 119 in a second configuration. In yet another embodiment, first and second buttons 107, 109 are operable to activate a first power level and a second power level of cutting element 106.

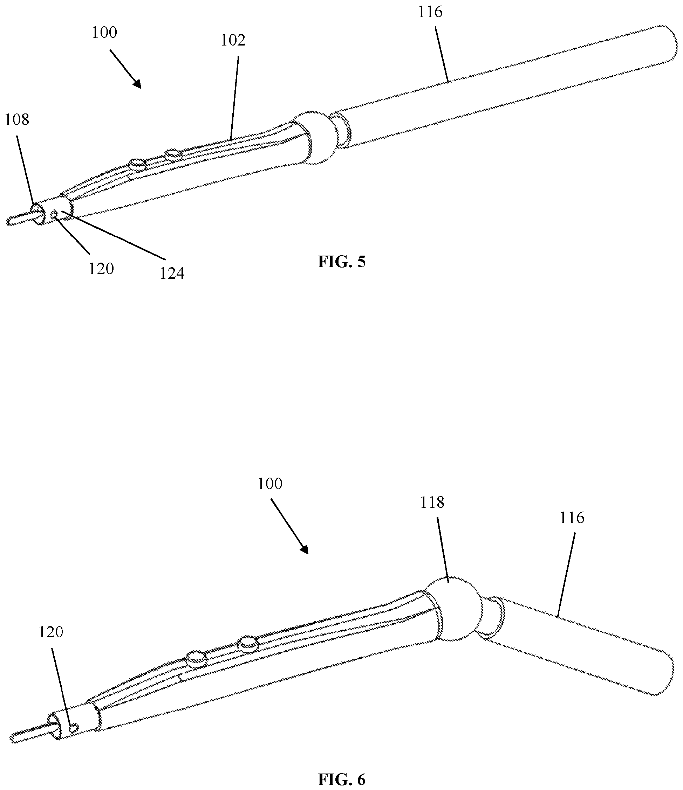

Adjacent cutting element 106 is inlet 108. Inlet 108 provides an opening that is fluidly connected to conduit 110. Conduit 110 extends through the longitudinal axis 103 of body 102. Conduit 110 provides a hollow channel that allows a flow of fluid, gas, and/or particulates through body 102. At the proximal end 112 of body 102 is outlet 114 (shown in FIG. 7 and FIG. 8). Outlet 114 is fluidly connected to conduit 110 and provides an opening that allows a flow of fluid, gas, and/or particulates to pass through it. Outlet 114 is fluidly coupled to tube 116. Tube 116 defines a hollow passage that is fluidly connected to outlet 114 and an evacuation source 117 (e.g., a vacuum source as shown in FIG. 2). Embodiments of evacuation source 117 are operable to urge or create a flow of fluid and/or particulates through inlet 108, perforations 120, conduit 110, outlet 114 and tube 116 such that a flow of fluid and/or particulates passes from inlet 108 to evacuation source 117. Embodiments of evacuation source 117 include a motor 119 and a power source 121 (e.g., power from an outlet or battery 123) such that evacuation source 117 is operable to create a vacuum or fluid flow through device 100 and tube 116. In this embodiment, outlet 114 is fluidly connected to ball swivel joint 118 having a hollow passageway 119 (see FIGs. 6-8) that is fluidly connected to tube 116, outlet 114 and conduit 110. Ball swivel joint 118 is operable to move and/or rotate to multiple angles allowing the tube 116 and/or body 102 to be moved relative to one another (see FIG. 1, FIG. 6, FIG. 7 and FIG. 8). Ball swivel 118 includes a socket portion that is rotatably coupled with body 102 adjacent outlet 114. The ball swivel 118 also includes a ball portion that is at least partially disposed within the socket portion such that the ball portion and the socket portion are in fluid communication with one another and together form hollow passageway 119.

Adjacent inlet 108 are perforations 120. Perforations 120 provide openings, passageways or holes in body 102 from a surrounding environment to conduit 110. Perforations 120 are in fluid communication (also referred to as fluidly connected) to conduit 110. Perforations 120 are operable to allow a flow of fluid, gas, and/or particulates from the surrounding environment through perforations 120 to conduit 110. It should be appreciated that embodiments of the present disclosure provide that outlet 114 allows a flow of fluid, gas and/or particulates at a first rate and perforations 120 allow a flow of at a second rate. In one embodiment the second rate with will less that the first rate. In another embodiment, a flow rate through perforations 120 is less than or equal to a flow rate of fluid through the outlet 114. Embodiments of perforations 120 define a gap having an area that is less than the area of inlet 108. Embodiments of perforations 120 in another embodiment define a gap having an area that is less than or equal to the area of inlet 108.

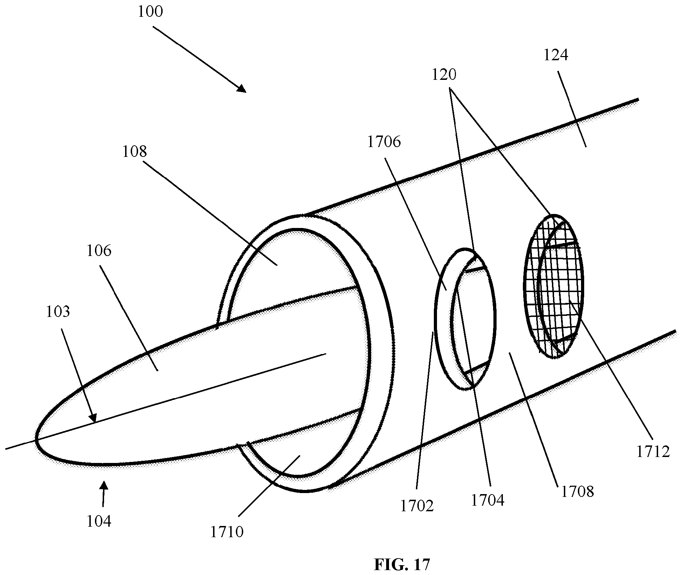

Reference is now made to FIG. 17, which depicts a close-up view of the distal end of device 100. Shown in FIG. 17 is electrode 106 extending from inlet 108 of shroud 124. Shroud 124 includes perforations 120, which provide gaps, holes or passageways to conduit 110. It should be appreciated that in this embodiment perforations 120 include an exterior opening portion 1702 (fluidly connected to the surrounding environment) adjacent the radial exterior surface 1708 of shroud 124, and an interior opening portion 1704 (fluidly connected to conduit 110) adjacent the radial interior surface 1710 of shroud 124. Exterior opening portion 1702 and interior opening portion 1704 of a perforation 120 are in fluid communication with one another and are connected by radial perforation surface 1706. The radial perforation surface 1706 is defined by the radial surface of shroud 124 that connects the exterior opening portion 1702 and the interior opening portion 1704. As depicted in FIG. 17, the exterior opening portion 1702 can be disposed closer to the distal end 104 than the interior opening portion 1704. In other words, the exterior opening portion 1702 and the interior opening portion 1704 do not run perpendicular the longitudinal axis 103, but are angled between 90 degrees and 10 degrees relative to the longitudinal axis 103. In other words, the radial perforation surface 1706 between the exterior opening portion 1702 and the interior opening portion 1704 can be angled toward the distal end 104 or any other direction. In another embodiment the exterior opening portion 1702 can be disposed closer to the proximal end 112 than the interior opening portion 1704 such that the radial perforation surface 1706 is angled toward the proximal end 112. In yet another embodiment, the exterior opening portion 1702 is disposed such that it is radially spaced from the interior opening portion 1704. Embodiments include some or all of the perforations 120 including a filter 1712 located within perforations 120 between the radial perforation surface 1706. Embodiments of filter 1712 are operable to remove gases, fluids and/or particulates from a flow that passes through filter 1712. Embodiments of filter 1712 include HEPA filters, carbon filters, and any type of air filter known in the art. Embodiments of carbon filters include porous carbon that is operable to trap or filter out particulates from gas and/or fluids that pass through perforations 120.

As shown in FIG. 1, device 100 includes three perforations 120 located adjacent inlet 108. However, it should be appreciated that embodiments provide there being one or multiple perforations 120 disposed anywhere along body 102. Referring to FIG. 2, shown is an embodiment of device 100 having two perforations 120 disposed adjacent inlet 108.

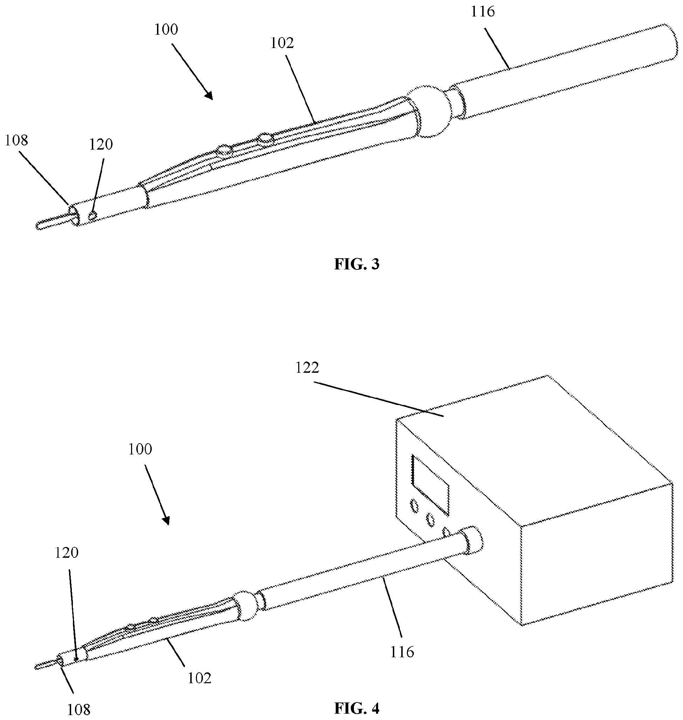

Referring to FIG. 3, shown is yet another embodiment of device 100. In this embodiment, device 100 includes one perforation 120 disposed adjacent inlet 108. Referring to FIG. 4, illustrated is device 100 with tube 116 coupled to filter assembly 122 operable to filter out gas, fluid and/or particulates from the flow that passes through inlet 108, perforations 120, conduit 110, outlet 114, and tube 116. It should be appreciated that embodiments include a vacuum or evacuation source being coupled to filter assembly 122 operable to urge or create a flow of fluid, gas, and/or particulates through inlet 108, perforations 120, conduit 110, outlet 114, and tube 116. Embodiments of a vacuum or evacuation source include a central vacuum unit installed in a wall of a medical facility, a standalone unit or a remote vacuum unit (as shown in FIG. 2) located adjacent to or spaced from the filter assembly 122.

Referring to FIG. 5, shown is yet another embodiment of device 100 having a body 102, an inlet 108 and one perforation 120. It should be appreciated in this embodiment that shroud 124 surrounding cutting element 106 has a length extending along the longitudinal axis 103 that is shorter than that found in FIGs. 1-3. Embodiments of device 100 include a shroud 124 partially extending over length of cutting element 106 by 10%, 25%, 50% or 75%.

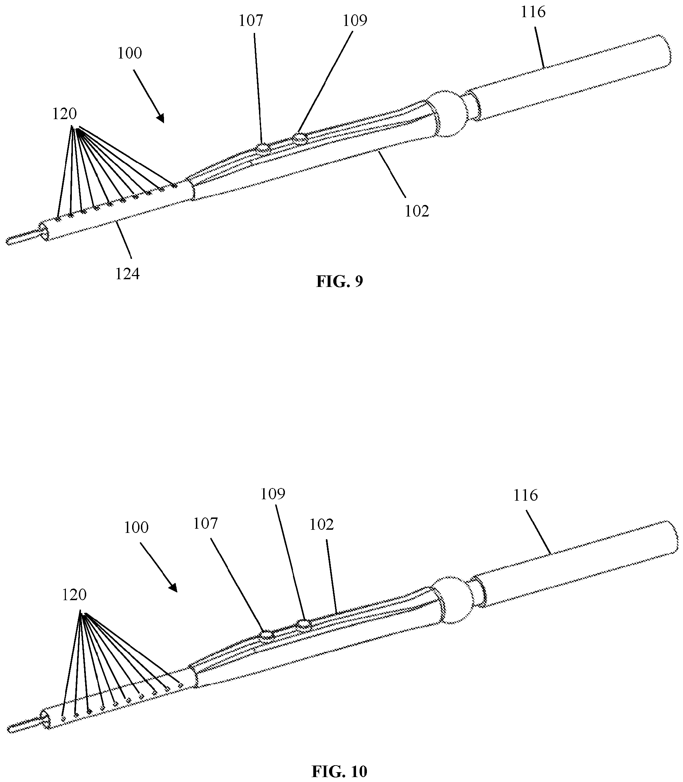

Reference is now made to FIG. 9, which illustrates another embodiment of device 100 having a body 102, a shroud 124 and perforations 120. It should be appreciated that embodiments provide that perforations 120 (as depicted in FIG. 9) are colinear with one another such that the perforations 120 are lined up adjacent to one another along the longitudinal axis 103 of device 100. In the embodiment shown in FIG. 9, perforations 120 are located along the same radial position with respect to the longitudinal axis 103 as first and second buttons 107, 109. However, it should be appreciated that embodiments of perforations 120 can be located colinear with one another at any radial location on body 102 (or shroud 124) or they can be non-colinear with one another. As shown in FIG. 10, perforations 120 are co-linear with one another along the longitudinal axis 103 of device 100, but are located 90 degrees from the radial location of first and second buttons 107, 109.

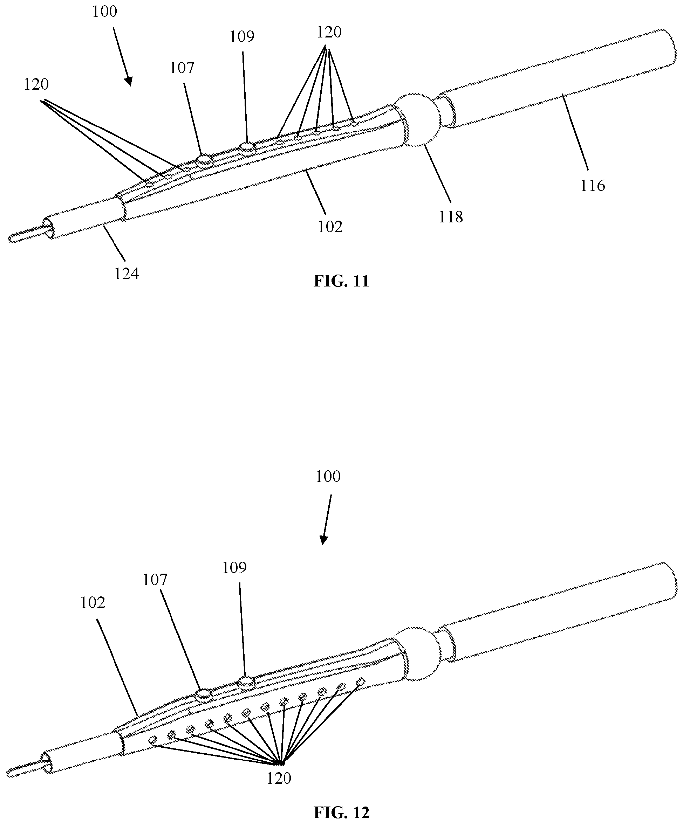

Referring to FIG. 11, shown is yet another exemplary embodiment of device 100 having a body 102 and perforations 120. As depicted in FIG. 11, perforations 120 are located along body 102 adjacent first and second buttons 107, 109 rather than the shroud 124 portion of body 102. In this embodiment, perforations 120 are colinear with first and second buttons 107, 109 along body 102 extending from adjacent shroud 124 to adjacent ball swivel 118. It should be appreciated that regardless of the location of perforations 120 on device 100, in each embodiment, perforations 120 provide a gap, hole or passageway between conduit 110 and the exterior environment of device 100. Referring to FIG. 12, shown is an embodiment of device 100 similar to that found in FIG. 11. Shown in FIG. 12 is device 100 having a body 102 and perforations 120 located colinear with one another along the longitudinal axis 103 of body 102. However, in this embodiment, perforations 120 are radially spaced from first and second buttons 107, 109 by approximately 90 degrees relative to the longitudinal axis 103.

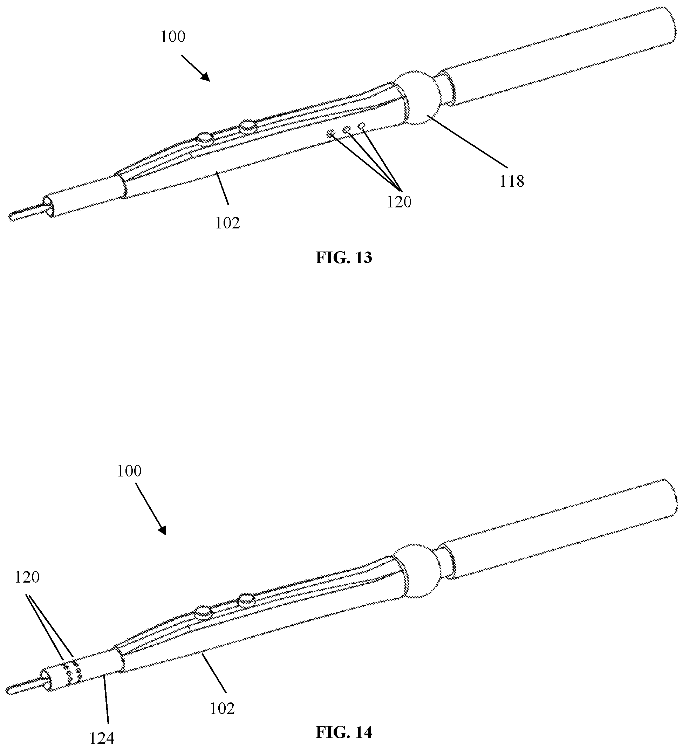

Referring to FIG. 13, shown is another exemplary embodiment of device 100 having a body 102 and perforations 120. In this embodiment, there are only three perforations 120 located adjacent ball swivel 118. It should be appreciated that embodiments of device 100 provide that perforations 120 are located on multiple radial locations (or sides) of device 100. For example, device 100 from FIG. 13 may include three perforations 120 along one side and three perforations 120 located along another side 180 degrees radially from the three perforations 120 depicted in FIG. 13.

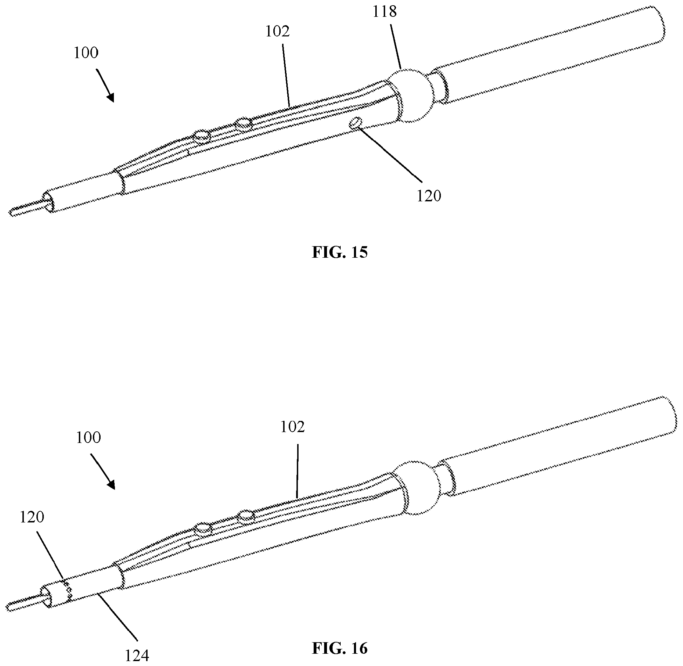

Reference is now made to FIG. 14, which depicts device 100 with body 102, shroud 124 and perforations 120. As shown in this embodiment, there are two sets of perforations 120 that are colinear with one another along the radial direction. In other words, perforations 120 circumscribe shroud 124 creating two rings around shroud 124. It should be appreciated that embodiments include perforations 120 forming one (shown in FIG. 16) or multiple rings around shroud 124 and/or body 102. Referring to FIG. 15, shown is another exemplary device 100 having a body 102 and a single perforation 120 located adjacent ball swivel 118.

In practice embodiments of device 100 can be used in surgical procedures. For instance, cutting element 106 will be used to cut human tissue. This cutting can cause the creation of surgical smoke or plume, which can include gases, fluids, and/or particulates to enter the atmosphere surrounding the surgical site. A vacuum source 117 fluidly connected to tube 116 and conduit 110 of device 100 will be activated such that a flow of gas, fluid and/or particulates is created or urged through inlet 108, perforations 120, conduit 110, outlet 114, and tube 116. Since the surgical smoke or plume may not be localized next to the surgical site, the inlet 108 alone will not be able to evacuate all of the visibility obstructing surgical smoke. In this regard, perforations 120 increase the area of evacuation such that the smoke or plume can be more quickly and efficiently removed from the surgical room/area. For the embodiment in which perforations 120 include filters 1712, gas, fluid, and/or particulates that pass through filters 1712 and perforations 120 will be filtered based on the type of filter 1712 located in each particular perforation 120.

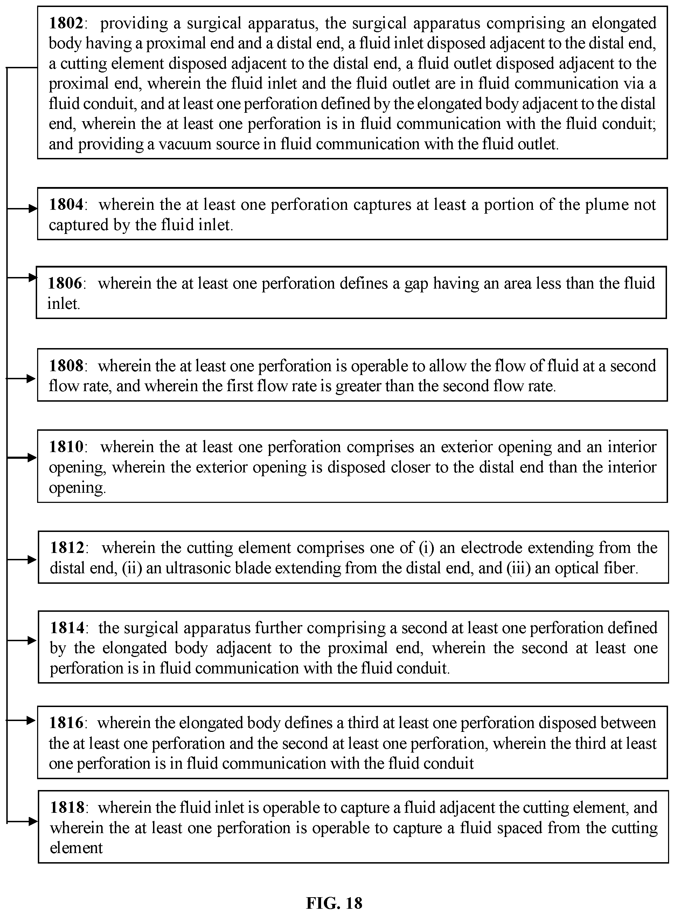

Reference is now made to FIG. 18, which depicts a logic flow diagram suitable for practicing exemplary embodiments of the present disclosure. Block 1802 states providing a surgical apparatus, the surgical apparatus comprising an elongated body having a proximal end and a distal end, a fluid inlet disposed adjacent to the distal end, a cutting element disposed adjacent to the distal end, a fluid outlet disposed adjacent to the proximal end, wherein the fluid inlet and the fluid outlet are in fluid communication via a fluid conduit, and at least one perforation defined by the elongated body adjacent to the distal end, wherein the at least one perforation is in fluid communication with the fluid conduit; and providing a vacuum source in fluid communication with the fluid outlet. Then block 1804 specifies wherein the at least one perforation captures at least a portion of the plume not captured by the fluid inlet.

Some of the non-limiting implementations detailed above are also summarized in FIG. 18 following block 1804. Block 1806 recites wherein the at least one perforation defines a gap having an area less than the fluid inlet. Block 1808 states wherein the at least one perforation is operable to allow the flow of fluid at a second flow rate, and wherein the first flow rate is greater than the second flow rate. Then block 1810 specifies wherein the at least one perforation comprises an exterior opening and an interior opening, wherein the exterior opening is disposed closer to the distal end than the interior opening. Block 1812 states wherein the cutting element comprises one of (i) an electrode extending from the distal end, (ii) an ultrasonic blade extending from the distal end, and (iii) an optical fiber. Next block 1814 relates to the surgical apparatus further comprising a second at least one perforation defined by the elongated body adjacent to the proximal end, wherein the second at least one perforation is in fluid communication with the fluid conduit. Then block 1816 indicates wherein the elongated body defines a third at least one perforation disposed between the at least one perforation and the second at least one perforation, wherein the third at least one perforation is in fluid communication with the fluid conduit. Finally, block 1818 states wherein the fluid inlet is operable to capture a fluid adjacent the cutting element, and wherein the at least one perforation is operable to capture a fluid spaced from the cutting element.

The logic diagram of FIG. 18 may be considered to illustrate the operation of a method, or a specific manner in which components of a device are configured to cause the device to operate or be provided, whether such device is an electrosurgical device, surgical device, or one or more components thereof.

This disclosure has been described in detail with particular reference to the above described embodiments, but it will be understood that variations and modifications can be effected. The presently disclosed embodiments are therefore considered in all respects to be illustrative and not restrictive. The scope of the invention is defined by the appended claims, and all changes that come within the meaning and range of equivalents thereof are intended to be embraced therein.

## Claims

1. A surgical apparatus, comprising:
an elongated body (102) comprising a longitudinal axis (103), a distal end (104), and a proximal end (112), the elongated body (102) comprising a fluid conduit (110) extending along the longitudinal axis (103);
a fluid inlet (108) disposed adjacent to the distal end (104), wherein the fluid inlet (108) is operable to allow a flow of fluid at a first flow rate;
a cutting element (106) disposed adjacent to the distal end (104);
a fluid outlet (114) disposed adjacent to the proximal end (112), wherein the fluid inlet (108) and the fluid outlet (114) are in fluid communication via the fluid conduit (110); and
at least one perforation (120) defined by the elongated body (102) adjacent to the distal end (104), wherein the at least one perforation (120) is in fluid communication with the fluid conduit (110);
**characterised in that** the at least one perforation (120) includes a filter (1712), wherein the filter is located within the perforation and is operable to remove gases, fluids and/or particles from a flow that passes through the filter.

2. The surgical apparatus according to claim 1, further comprising:
a swivel portion (118) rotatably coupled with the elongated body (102) adjacent to the fluid outlet (114), wherein the swivel portion (118) includes a socket in fluid communication with the fluid conduit (110);
a ball portion at least partially disposed within the socket, wherein the ball portion is in fluid communication with the socket; and
a second fluid conduit coupled with the ball portion, wherein the second fluid conduit is in fluid communication with the ball portion.

3. The surgical apparatus according to claim 1, wherein the at least one perforation (120) defines a gap having an area less than the fluid inlet (108).

4. The surgical apparatus according to claim 1, wherein the at least one perforation (120) is operable to allow the flow of fluid at a second flow rate, and wherein the first flow rate is greater than the second flow rate.

5. The surgical apparatus according to claim 1, wherein the at least one perforation (120) comprises an exterior opening (1702) and an interior opening (1704), wherein the exterior opening (1702) is disposed closer to the distal end (104) than the interior opening (1704).

6. The surgical apparatus according to claim 1, wherein the cutting element (106) comprises an electrode extending from the distal end(104).

7. The surgical apparatus according to claim 1, wherein the cutting element (106) comprises an ultrasonic blade extending from the distal end (104).

8. The surgical apparatus according to claim 1, wherein the cutting element (106) comprises an optical fiber.

9. The surgical apparatus according to claim 2, further comprising:
a vacuum tube (116) fluidly coupled with the fluid conduit (110); and
a vacuum source (117) fluidly coupled with the vacuum tube (116), the vacuum source (117) operable to create a flow of fluid.

10. The surgical apparatus according to claim 1, further comprising:
a first button and a second button (107, 109) coupled to the elongated body (102), wherein the first and second button(107, 109) are operable to control the cutting element (106);
wherein preferably the first button (107) is operable to activate a first power level; and the second button (109) is operable to activate a second power level.

11. The surgical apparatus according to claim 1, further comprising:
a second at least one perforation (120) defined by the elongated body (102) adjacent to the proximal end (112), wherein the second at least one perforation (120) is in fluid communication with the fluid conduit (110).

12. The surgical apparatus according to claim 11, wherein the elongated body (102) defines a third at least one perforation (120) disposed between the at least one perforation (120) and the second at least one perforation (120), wherein the third at least one perforation (120) is in fluid communication with the fluid conduit (110).

13. The surgical apparatus according to claim 1, wherein the fluid inlet (108) is operable to capture a fluid adjacent the cutting element (106), and wherein the at least one perforation (120) is operable to capture a fluid spaced from the cutting element (106).

14. The surgical apparatus according to claim 1, comprising:
a plurality of perforations (120) defined by the elongated body (102) located adjacent the distal end (104), wherein the plurality of perforations (120) are in fluid communication with the fluid conduit (110);
wherein preferably the plurality of perforations (120) defined by the elongated body (102) are located along the length thereof between the distal end (104) and the proximal end (112); and
wherein further preferably the elongated body (102) defines a top portion and a bottom portion; and the plurality of perforations (120) is located through the top portion.

15. A method of providing a surgical apparatus (100) according to any one of claims 1 to 14, the method comprising:
(a) providing a surgical apparatus (100), the surgical apparatus (100) comprising:
an elongated body (102) having a proximal end (112) and a distal end (104), a fluid inlet (108) disposed adjacent to the distal end (104), a cutting element (106) disposed adjacent to the distal end (104);
a fluid outlet (114) disposed adjacent to the proximal end (112), wherein the fluid inlet (108) and the fluid outlet (114) are in fluid communication via a fluid conduit (110); and
at least one perforation (120) defined by the elongated body (102) adjacent to the distal end (104), wherein the at least one perforation (120) is in fluid communication with the fluid conduit (110); and
(b) providing a vacuum source (117) in fluid communication with the fluid outlet (114);
**characterised in that** in step (a) the at least one perforation (120) includes a filter (1712), wherein the filter is located within the perforation and is operable to remove gases, fluids and/or particles from a flow that passes through the filter.

16. The method according to claim 15, wherein the at least one perforation (120) captures at least a portion of plume not captured by the fluid inlet (108);
wherein preferably the at least one perforation (120) comprises an exterior opening (1702) and an interior opening (1704), wherein the exterior opening (1702) is disposed closer to the distal end (104) than the interior opening (1704).

## Patentansprüche

1. Chirurgische Vorrichtung, die Folgendes umfasst:
einen länglichen Körper (102), der eine Längsachse (103), ein distales Ende (104) und ein proximales Ende (112) aufweist, wobei der längliche Körper (102) eine Fluidleitung (110) aufweist, die sich entlang der Längsachse (103) erstreckt;
einen Fluideinlass (108), der angrenzend an das distale Ende (104) angeordnet ist, wobei der Fluideinlass (108) betreibbar ist, um einen Fluidstrom mit einer ersten Durchflussrate zu ermöglichen;
ein Schneidelement (106), das angrenzend an das distale Ende (104) angeordnet ist;
einen Fluidauslass (114), der angrenzend an das proximale Ende (112) angeordnet ist, wobei der Fluideinlass (108) und der Fluidauslass (114) über die Fluidleitung (110) in Fluidverbindung stehen; und
mindestens eine Perforation (120), die durch den länglichen Körper (102) angrenzend an das distale Ende (104) gebildet ist, wobei die mindestens eine Perforation (120) in Fluidverbindung mit der Fluidleitung (110) steht;
**dadurch gekennzeichnet, dass** die mindestens eine Perforation (120) einen Filter (1712) aufweist, wobei der Filter innerhalb der Perforation angeordnet ist und dazu dient, Gase, Flüssigkeiten und/oder Partikel aus einem Strom zu entfernen, der durch den Filter strömt.

2. Chirurgische Vorrichtung nach Anspruch 1, die ferner umfasst:
einen Drehabschnitt (118), der drehbar mit dem länglichen Körper (102) angrenzend an den Fluidauslass (114) gekoppelt ist, wobei der Drehabschnitt (118) eine Pfanne in Fluidverbindung mit der Fluidleitung (110) aufweist;
einen Kugelabschnitt, der zumindest teilweise innerhalb der Pfanne angeordnet ist, wobei der Kugelabschnitt in Fluidverbindung mit der Pfanne steht; und
eine zweite Fluidleitung, die mit dem Kugelabschnitt gekoppelt ist, wobei die zweite Fluidleitung in Fluidverbindung mit dem Kugelabschnitt steht.

3. Chirurgische Vorrichtung nach Anspruch 1, wobei die mindestens eine Perforation (120) einen Spalt mit einer Fläche bildet, die kleiner ist als der Fluideinlass (108).

4. Chirurgische Vorrichtung nach Anspruch 1, wobei die mindestens eine Perforation (120) so betreibbar ist, dass sie den Durchfluss von Flüssigkeit mit einer zweiten Durchflussrate ermöglicht, und wobei die erste Durchflussrate größer ist als die zweite Durchflussrate.

5. Chirurgische Vorrichtung nach Anspruch 1, wobei die mindestens eine Perforation (120) eine äußere Öffnung (1702) und eine innere Öffnung (1704) aufweist, wobei die äußere Öffnung (1702) näher am distalen Ende (104) angeordnet ist als die innere Öffnung (1704).

6. Chirurgische Vorrichtung nach Anspruch 1, wobei das Schneidelement (106) eine Elektrode umfasst, die sich vom distalen Ende (104) aus erstreckt.

7. Chirurgische Vorrichtung nach Anspruch 1, wobei das Schneidelement (106) eine Ultraschallklinge umfasst, die sich vom distalen Ende (104) aus erstreckt.

8. Chirurgische Vorrichtung nach Anspruch 1, wobei das Schneidelement (106) eine optische Faser umfasst.

9. Chirurgische Vorrichtung nach Anspruch 2, die ferner umfasst:
eine Vakuumröhre (116), die mit der Flüssigkeitsleitung (110) verbunden ist; und
eine Vakuumquelle (117), die mit der Vakuumröhre (116) in Fluidverbindung steht, wobei die Vakuumquelle (117) einen Fluidstrom erzeugen kann.

10. Chirurgische Vorrichtung nach Anspruch 1, die ferner umfasst:
eine erste Taste und eine zweite Taste (107, 109), die mit dem länglichen Körper (102) gekoppelt sind, wobei die erste und die zweite Taste (107, 109) betätigbar sind, um das Schneidelement (106) zu steuern;
wobei vorzugsweise die erste Taste (107) zur Aktivierung einer ersten Leistungsstufe und die zweite Taste (109) zur Aktivierung einer zweiten Leistungsstufe betätigt werden kann.

11. Chirurgische Vorrichtung nach Anspruch 1, die ferner umfasst:
mindestens eine zweite Perforation (120), die durch den länglichen Körper (102) angrenzend an das proximale Ende (112) gebildet ist, wobei die mindestens zweite Perforation (120) in Fluidverbindung mit der Fluidleitung (110) steht.

12. Chirurgische Vorrichtung nach Anspruch 11, wobei der längliche Körper (102) mindestens eine dritte Perforation (120) bildet, die zwischen der mindestens einen Perforation (120) und der mindestens zweiten Perforation (120) angeordnet ist, wobei die mindestens dritte Perforation (120) in Fluidverbindung mit der Fluidleitung (110) steht.

13. Chirurgische Vorrichtung nach Anspruch 1, wobei der Fluideinlass (108) zum Auffangen eines Fluids in der Nähe des Schneidelements (106) betreibbar ist, und wobei die mindestens eine Perforation (120) zum Auffangen eines Fluids in einem Abstand von dem Schneidelement (106) betreibbar ist.

14. Chirurgische Vorrichtung nach Anspruch 1, umfassend:
eine Vielzahl von Perforationen (120), die durch den länglichen Körper (102) gebildet sind und sich in der Nähe des distalen Endes (104) befindet, wobei die Vielzahl von Perforationen (120) in Fluidverbindung mit der Fluidleitung (110) steht;
wobei vorzugsweise die Vielzahl von Perforationen (120), die durch den länglichen Körper (102) gebildet ist, entlang dessen Länge zwischen dem distalen Ende (104) und dem proximalen Ende (112) angeordnet sind; und
wobei ferner vorzugsweise der längliche Körper (102) einen oberen Abschnitt und einen unteren Abschnitt bildet; und die Vielzahl von Perforationen (120) durch den oberen Abschnitt angeordnet ist.

15. Verfahren zur Bereitstellung einer chirurgischen Vorrichtung (100) nach einem der Ansprüche 1 bis 14, wobei das Verfahren umfasst:
(a) Bereitstellen einer chirurgischen Vorrichtung (100), wobei die chirurgische Vorrichtung (100) umfasst:
einen länglichen Körper (102) mit einem proximalen Ende (112) und einem distalen Ende (104), einen Fluideinlass (108), der angrenzend an das distale Ende (104) angeordnet ist, ein Schneidelement (106), das angrenzend an das distale Ende (104) angeordnet ist;
einen Fluidauslass (114), der angrenzend an das proximale Ende (112) angeordnet ist, wobei der Fluideinlass (108) und der Fluidauslass (114) über eine Fluidleitung (110) in Fluidverbindung stehen; und
mindestens eine Perforation (120), die durch den länglichen Körper (102) angrenzend an das distale Ende (104) gebildet ist, wobei die mindestens eine Perforation (120) in Fluidverbindung mit der Fluidleitung (110) steht; und
(b) Bereitstellen einer Vakuumquelle (117) in Fluidverbindung mit dem Fluidauslass (114);
**dadurch gekennzeichnet, dass** in Schritt (a) die mindestens eine Perforation (120) einen Filter (1712) aufweist, wobei der Filter innerhalb der Perforation angeordnet ist und dazu dient, Gase, Flüssigkeiten und/oder Partikel aus einem Strom zu entfernen, der durch den Filter strömt.

16. Verfahren nach Anspruch 15, wobei die mindestens eine Perforation (120) mindestens einen Teil der vom Fluideinlass (108) nicht erfassten Abluft einfängt;
wobei vorzugsweise die mindestens eine Perforation (120) eine äußere Öffnung (1702) und eine innere Öffnung (1704) aufweist, wobei die äußere Öffnung (1702) näher am distalen Ende (104) angeordnet ist als die innere Öffnung (1704).

## Revendications

1. Un appareil chirurgical comprenant:
un corps allongé (102) comprenant un axe longitudinal (103), une extrémité distale (104) et une extrémité proximale (112), le corps allongé (102) comprenant un conduit de fluide (110) s'étendant le long de l'axe longitudinal (103) ;
une entrée de fluide (108) disposée à côté de l'extrémité distale (104), dans laquelle l'entrée de fluide (108) peut être utilisée pour permettre un écoulement de fluide à un premier débit ;
un élément de coupe (106) disposé à côté de l'extrémité distale (104) ;
une sortie de fluide (114) disposée à côté de l'extrémité proximale (112), dans laquelle l'entrée de fluide (108) et la sortie de fluide (114) sont en communication fluidique par l'intermédiaire du conduit de fluide (110) ; et
au moins une perforation (120) définie par le corps allongé (102) adjacent à l'extrémité distale (104), dans laquelle au moins une perforation (120) est en communication fluidique avec le conduit fluidique (110) ;
**caractérisé par le fait que** l'au moins une perforation (120) comprend un filtre (1712), dans lequel le filtre est situé à l'intérieur de la perforation et est capable d'éliminer les gaz, les fluides et/ou les particules d'un flux qui passe à travers le filtre.

2. L'appareil chirurgical selon la revendication 1, comprenant en outre :
une partie pivotante (118) couplée de manière rotative au corps allongé (102) adjacent à la sortie de fluide (114), dans laquelle la partie pivotante (118) comprend une douille en communication fluide avec le conduit de fluide (110) ;
une partie sphérique au moins partiellement disposée à l'intérieur de la douille, dans laquelle la partie sphérique est en communication fluide avec la douille ; et
un second conduit de fluide couplé à la partie sphérique, dans lequel le second conduit de fluide est en communication fluide avec la partie sphérique.

3. L'appareil chirurgical selon la revendication 1, dans lequel l'au moins une perforation (120) définit un espace ayant une surface inférieure à l'entrée de fluide (108).

4. L'appareil chirurgical selon la revendication 1, dans lequel au moins une perforation (120) peut permettre l'écoulement du fluide à un second débit, et dans lequel le premier débit est supérieur au second débit.

5. L'appareil chirurgical selon la revendication 1, dans lequel au moins une perforation (120) comprend une ouverture extérieure (1702) et une ouverture intérieure (1704), l'ouverture extérieure (1702) étant disposée plus près de l'extrémité distale (104) que l'ouverture intérieure (1704).

6. L'appareil chirurgical selon la revendication 1, dans lequel l'élément de coupe (106) comprend une électrode s'étendant à partir de l'extrémité distale (104).

7. L'appareil chirurgical selon la revendication 1, dans lequel l'élément de coupe (106) comprend une lame ultrasonique s'étendant à partir de l'extrémité distale (104).

8. L'appareil chirurgical selon la revendication 1, dans lequel l'élément de coupe (106) comprend une fibre optique.

9. L'appareil chirurgical selon la revendication 2, comprenant en outre :
un tube à vide (116) couplé de manière fluide au conduit de fluide (110) ; et
une source de vide (117) couplée fluidiquement au tube à vide (116), la source de vide (117) pouvant être utilisée pour créer un flux de fluide.

10. L'appareil chirurgical selon la revendication 1, comprenant en outre :
un premier bouton et un second bouton (107, 109) couplés au corps allongé (102), le premier et le second bouton (107, 109) pouvant commander l'élément de coupe (106) ;
où, de préférence, le premier bouton (107) permet d'activer un premier niveau de puissance ; et le second bouton (109) permet d'activer un second niveau de puissance.

11. L'appareil chirurgical selon la revendication 1, comprenant en outre :
une deuxième perforation (120) au moins, définie par le corps allongé (102) adjacent à l'extrémité proximale (112), dans laquelle la deuxième perforation (120) au moins est en communication fluidique avec le conduit de fluide (110).

12. L'appareil chirurgical selon la revendication 11, dans lequel le corps allongé (102) définit une troisième perforation au moins (120) disposée entre la perforation au moins (120) et la deuxième perforation au moins (120), dans laquelle la troisième perforation au moins (120) est en communication fluidique avec le conduit fluidique (110).

13. L'appareil chirurgical selon la revendication 1, dans lequel l'entrée de fluide (108) est apte à capturer un fluide adjacent à l'élément de coupe (106), et dans lequel l'au moins une perforation (120) est apte à capturer un fluide espacé de l'élément de coupe (106).

14. L'appareil chirurgical selon la revendication 1, comprenant :
une pluralité de perforations (120) définies par le corps allongé (102) situé à proximité de l'extrémité distale (104), la pluralité de perforations (120) étant en communication fluidique avec le conduit fluidique (110) ;
dans lequel, de préférence, la pluralité de perforations (120) définies par le corps allongé (102) est située sur sa longueur entre l'extrémité distale (104) et l'extrémité proximale (112) ; et
où, de préférence, le corps allongé (102) définit une partie supérieure et une partie inférieure ; et la pluralité de perforations (120) est située à travers la partie supérieure.

15. Méthode de fourniture d'un appareil chirurgical (100) selon l'une quelconque des revendications 1 à 14, la méthode comprenant :
(a) fournir un appareil chirurgical (100), l'appareil chirurgical (100) comprenant :
un corps allongé (102) ayant une extrémité proximale (112) et une extrémité distale (104), une entrée de fluide (108) disposée à côté de l'extrémité distale (104), un élément de coupe (106) disposé à côté de l'extrémité distale (104) ;
une sortie de fluide (114) disposée à côté de l'extrémité proximale (112), dans laquelle l'entrée de fluide (108) et la sortie de fluide (114) sont en communication fluidique par l'intermédiaire d'un conduit de fluide (110) ; et
au moins une perforation (120) définie par le corps allongé (102) adjacent à l'extrémité distale (104), dans laquelle au moins une perforation (120) est en communication fluidique avec le conduit fluidique (110) ; et
(b) fournir une source de vide (117) en communication fluide avec la sortie de fluide (114) ;
**caractérisé par le fait qu'**à l'étape (a), au moins une perforation (120) comprend un filtre (1712), dans lequel le filtre est situé à l'intérieur de la perforation et est capable d'éliminer les gaz, les fluides et/ou les particules d'un flux qui passe à travers le filtre.

16. Méthode selon la revendication 15, dans laquelle au moins une perforation (120) capture au moins une partie du panache non capturé par l'entrée de fluide (108) ;
où, de préférence, l'au moins une perforation (120) comprend une ouverture extérieure (1702) et une ouverture intérieure (1704), l'ouverture extérieure (1702) étant disposée plus près de l'extrémité distale (104) que l'ouverture intérieure (1704).
